# EUROPÄISCHE PATENTANMELDUNG

(11) **EP 4 575 502 A1**
(43) Veröffentlichungstag der Anmeldung: **25.06.2025**
(21) Anmeldenummer: 24220866.8
(22) Anmeldetag: 18.12.2024
(51) Int. Cl.: G01N 33/543

(54) **VORRICHTUNG FÜR EINEN LATERAL-FLOW-TEST MIT BELÜFTUNG UND/ODER AMYLASE-INDIKATOR**

(30) Priorität: 18.12.2023 AT 510142023
(71) Anmelder: Tagtron GmbH, 2340 Mödling (AT)
(72) Erfinder: MITTERNDORFER, Fridolin, 4844 Regau (AT); SCHEFZIG, Fabian, 4844 Regau (AT); SCHODERMAYR, Christoph, 4844 Regau (AT)
(74) Vertreter: SONN Patentanwälte GmbH & Co KG

(57) **Zusammenfassung**

Die Erfindung betrifft eine Vorrichtung (1) für einen Lateral-Flow-Test aufweisend: ein Gehäuse (2), welches eine Probeneintrittsöffnung (3) hat, eine an die Probeneintrittsöffnung (3) angrenzende Vorkammer (4), ein mit der Vorkammer (4) verbundenes Probekissen (5), ein Konjugatkissen (8), welches mit dem Probekissen (5) in Verbindung steht, einen Laufstreifen (11), welcher mit dem Konjugatkissen (8) an einem Ende des Laufstreifens (11) in Verbindung steht, ein Saugkissen (14), welches mit dem Laufstreifen (11) an einem weiteren Ende des Laufstreifens (11) in Verbindung steht; wobei das Saugkissen ein Amylasesubstrat aufweist, wobei das Amylasesubstrat durch Amylase abgebaut werden kann.

## Beschreibung

### Hintergrund der Erfindung

Zum qualitativen Nachweis von Stoffen mittels Antigen-Antikörper Reaktion werden Lateral-Flow-Tests angewendet. Diese Tests sind eine Kombination aus einer Dünnschichtchromatographie und einer Immunfärbung. Zur Erreichung eines geringeren Geräteaufwandes werden Lateral-Flow-Tests oftmals in Form von Teststreifen, z.B. bei Schwangerschaftsteststreifen, Drogenteststreifen oder Allergenschnelltests verwendet. Bloße Teststreifen sind aber in der Handhabung oftmals fehleranfällig.

Eine Möglichkeit, Fehler des Lateral-Flow-Tests zu reduzieren, ist beispielsweise die Detektion von Kontrollanalyten wie in der Publikation WO 2020/237308 A1, der WO 2021/231659 A1 oder der US 2021/325383 A1 beschrieben.

In AT 17585 U1 wird ein rohrförmiger Lateral-Flow-Test beschrieben.

In WO 2016/164365 A1 betrifft eine Vorrichtung für einen Lateral-Flow-Test mit einem aus einem Gehäuse ragenden Mundstück.

WO 01/55723 A1 beschreibt eine Vorrichtung für einen Immunoassay bei dem eine Probe auf einen Probeaufnahmebereich aufgetropft wird.

US 2023/122646 A1 beschreibt eine stiftförmigen Antigendetektor, der einen Schaum hat, der mit einer Probe getränkt wird und mit dem inneren des Detektors verbunden ist.

Die bekannten Lateral-Flow-Tests sind für die Testung von Covid-19 dergestalt aufgebaut, dass zumeist nach Entfernung einer Schutzkappe der Anwender Speichel auf einem Absorptionsmaterial sammeln muss. Als Absorptionsmaterial kommen regelmäßig Filzlagen zur Anwendung. Der Benutzer muss bei der Anwendung das Absorptionsmaterial mit seinem Speichel feucht machen und muss das Absorptionsmaterial dazu in den Mund nehmen. Dieses "in den Mund nehmen" der Filzlagen wird von den Anwendern als nicht angenehm empfunden. Darüber hinaus nimmt das Absorptionsmaterial den Speichel nur langsam auf, sodass es eine etwas längere Zeit dauert, bis das Absorptionsmaterial des Lateral-Flow-Tests aus dem Mund genommen werden kann.

Die Erfindung hat sich daher die Ziele gesetzt, einen Lateral-Flow-Test zu entwickeln, der sowohl kostengünstig produziert werden kann als auch leicht zu entsorgen ist und überdies bei der Anwendung durch den Benutzer nicht als unangenehm empfunden wird.

Es ist ein weiteres Ziel der Erfindung Fehler im Lateral-Flow-Test, die insbesondere durch unterschiedliche Handhabung entstehen, zu reduzieren oder zu beseitigen. Um bei Massentests gut eingesetzt werden zu können, ist es weiters notwendig, dass die Anwendung des Lateral-Flow-Tests einerseits rasch durchgeführt werden kann und andererseits mit keinen oder möglichst geringen Unannehmlichkeiten für den Anwender verbunden ist.

### Zusammenfassung der Erfindung

Die Erfindung betrifft Lateral-Flow-Test mit einem Gehäuse und einer Vorkammer im Gehäuse, wobei das Gehäuse im Bereich der Vorkammer eine oder mehrere Lüftungsöffnung hat.

Konkret betrifft die Erfindung eine Vorrichtung (1) für einen Lateral-Flow-Test aufweisend: ein Gehäuse (2), welches eine Probeneintrittsöffnung (3) hat, eine an die Probeneintrittsöffnung (3) angrenzende Vorkammer (4), ein mit der Vorkammer (4) verbundenes Probekissen (5), ein Konjugatkissen (8), welches mit dem Probekissen (5) in Verbindung steht, einen Laufstreifen (11), welcher mit dem Konjugatkissen (8) an einem Ende des Laufstreifens (11) in Verbindung steht, ein Saugkissen (14), welches mit dem Laufstreifen (11) an einem weiteren Ende des Laufstreifens (11) in Verbindung steht; wobei das Gehäuse im Bereich der Vorkammer von der Probeneintrittsöffnung aus gesehen der Länge nach in ihrem letzten Drittel vor dem Probekissen (5) und/oder im Bereich des Probekissens (5) mindestens eine mit der Vorkammer in Verbindung stehende Lüftungsöffnung (16) hat.

Weiters betrifft die Erfindung eine Vorrichtung (1) für einen Lateral-Flow-Test aufweisend: ein Gehäuse (2), welches eine Probeneintrittsöffnung (3) hat, eine an die Probeneintrittsöffnung (3) angrenzende Vorkammer (4), ein mit der Vorkammer (4) verbundenes Probekissen (5), ein Konjugatkissen (8), welches mit dem Probekissen (5) in Verbindung steht, einen Laufstreifen (11), welcher mit dem Konjugatkissen (8) an einem Ende des Laufstreifens (11) in Verbindung steht, ein Saugkissen (14), welches mit dem Laufstreifen (11) an einem weiteren Ende des Laufstreifens (11) in Verbindung steht; wobei das Saugkissen ein Amylasesubstrat aufweist.

Weiters betrifft die Erfindung ein Verfahren zur Bestimmung eines Analyten, umfassend Aufbringen einer flüssigen Probe in die Vorkammer (4) der Vorrichtung, wobei die flüssige Probe durch das Probekissen (5) aufgesogen wird und durch weitere Sogwirkungen des damit verbundenen Konjugatkissens (8), des damit verbundenen Laufstreifens (11) und des damit verbundenen Saugkissens (14) zumindest teilweise durch das Konjugatkissen (8), durch den Laufstreifen (11), zum Saugkissen (14) befördert wird, wobei in einem Testbereich (12) am Laufstreifen (11) der Analyt gebunden wird.

Weiters betrifft die Erfindung ein Herstellungsverfahren zur Produktion einer Vorrichtung gemäß der Erfindung, umfassend Einbringen des Probekissens (5), des Konjugatkissens (8), des Laufstreifens (11) und des Saugkissens (14) in das Gehäuse (2) mit der Probeneintrittsöffnung (3). Das Gehäuse kann mindestens eine Lüftungsöffnung (16) aufweisen, wobei das Gehäuse (2) vorzugswese im Bereich der Vorkammer (4) die mindestens eine Lüftungsöffnung (16) hat. Das Saugkissen kann ein Amylasesubstrat aufweisen.

Alle Aspekte der Erfindung sind miteinander verbunden und die detaillierte Beschreibung eines Aspekts der Erfindung betrifft ebenso die anderen Aspekte. Z.B. kann jede Vorrichtung der Erfindung im Verfahren zur Bestimmung eines Analyten eingesetzt werden oder die Vorrichtung kann eine Eignung zu oder die Mittel für ein beschriebenes Verfahren zur Bestimmung eines Analyten haben. Jede beschriebene Vorrichtung kann das Resultat des Herstellungsverfahrens sein. Merkmale des Herstellungsverfahrens können in der Vorrichtung zu finden sein. Die Vorrichtung kann nach jedem der beschriebenen Herstellungsverfahren erhältlich sein.

### Figuren

Figur 1 zeigt eine perspektivische Explosionsansicht der in der Vorrichtung einsetzbaren Einzelteile.
Figur 2 zeigt eine perspektivische Ansicht des Innenlebens der Vorrichtung; oben mit Probekissen (5) und einem Saugkissen (14); unten ohne diese Teile und nur Teile, die am Träger (6) direkt aufliegen. Hier ist der optionale Indikatorbereich (15) nicht gezeigt.
Figur 3 zeigt Seitenansichten der Vorrichtung, a. Aufsicht auf die Vorrichtung mit Gehäuse (2); b. Aufsicht auf das Innenleben im Gehäuse (2); c. Längsquerschnitt auf die Vorrichtung mit Gehäuse (2); d. Längsquerschnitt auf das Innenleben im Gehäuse (2); e. Längsquerschnitt auf das Innenleben im Gehäuse (2) ohne Probekissen (5).

### Detaillierte Beschreibung der Erfindung

Die Erfindung betrifft eine Vorrichtung für einen Lateral-Flow-Test aufweisend: ein Gehäuse, welches eine Probeneintrittsöffnung hat, eine an die Probeneintrittsöffnung angrenzende Vorkammer, ein mit der Vorkammer verbundenes Probekissen, ein Konjugatkissen, welches mit dem Probekissen in Verbindung steht, einen Laufstreifen, welcher mit dem Konjugatkissen an einem Ende des Laufstreifens in Verbindung steht, ein Saugkissen, welches mit dem Laufstreifen an einem weiteren Ende des Laufstreifens in Verbindung steht.

Gemäß einer Ausführungsform der Erfindung hat das Gehäuse im Bereich der Vorkammer von der Probeneintrittsöffnung aus gesehen der Länge nach in ihrem letzten Drittel vor dem Probekissen und/oder im Bereich des Probekissens mindestens eine mit der Vorkammer in Verbindung stehende Lüftungsöffnung. Gemäß einer weiteren, mit der vorgenannten Ausführungsform kombinierbaren, Ausführungsform hat das Saugkissen ein Amylasesubstrat. Das Amylasesubstrat kann als Indikator für das Enzym Amylase dienen, welches das Amylasesubstrat abbauen kann. Amylase kann in einer Speichelprobe vorkommen, womit das Amylasesubstrat indirekt ein Indikator für Speichel oder einer konkreten Menge davon, basierend auf der Amylaseaktivität, ist.

Die Lüftungsöffnung im Bereich der Vorkammer oder des Probekissens bewirken, dass Luft bei zu geringer in die Vorkammer eingebrachter Probeflüssigkeit Luft entweichen kann und keine blockierenden Luftblasen in der Vorkammer entstehen. Somit kann die gesamte Probeflüssigkeit für den Test nutzbar gemacht werden, und insbesondre durch ein Probekissen nach der Vorkammer aufgenommen werden.

Eine Probenaufnahme direkt in das Probekissen ist nicht zielführend, denn dieses kann nicht die gesamte Probeflüssigkeit schnell genug aufnehmen, wodurch Probeflüssigkeit verloren gehen würde. Daher wird die Vorkammer vorgesehen. Von der Vorkammer kann die Probe teilweise oder auch vollständig in das Probekissen aufgenommen werden.

Die Lüftungsöffnung hat weiters den Vorteil, dass im Fall von zu viel aufgebrachter Probeflüssigkeit, diese durch Lüftungsöffnung aus dem Gehäuse ausgebracht werden kann.

Das Ende des Gehäuses mit der Probeneintrittsöffnung kann von einem Benutzer direkt in den Mund genommen werden und Probenflüssigkeit vom Mund des Benutzers direkt in die Vorkammer eingebracht werden. Manche Benutzer neigen dazu, bei diesem Vorgang zu Pusten, was in der Vorkammer zu hohem Druck und ggf. zu Beschädigung der Vorrichtung führen könnte, z.B. durch Verschieben des Probekissens und damit verbundener Elemente. Durch die Lüftungsöffnung, insbesondere der Lüftungsöffnung in der Vorkammer, zu einem Teil auch durch eine Lüftungsöffnung im Bereich des Probekissens, kann diese Gefahr reduziert werden, denn Druck durch Pusten kann sich aufgrund von Ausströmen von Luft oder Probenflüssigkeit durch die Lüftungsöffnung nicht aufbauen bzw. wird der Überdruck durch Entlüften abgebaut.

Manche Benutzer neigen dazu nach oder vor Einbringen der Probeflüssigkeit in die Vorkammer an der Probeneintrittsöffnung Anzusaugen. Dies könnte dazu führen, dass Probe wieder entfernt wird, ggf. sogar nach Aufnahme im Probekissen, in dem Reagenzien gelöst werden können. Ebenso könnten Beschädigungen an der Vorrichtung durch den Unterdruck durch Ansaugen entstehen. Auch diese Gefahren werden durch die Lüftungsöffnung reduziert, denn ein Unterdruck kann sich nicht oder schwerer im Gehäuse aufbauen, denn Luft kann zum Druckausgleich von der Umgebung durch die Lüftungsöffnung in das Gehäuse einströmen.

Diese beiden Fallkonstellationen zusammen bewirken also eine Normierung der verwertbaren Probeflüssigkeit, welche dem Test und zunächst dem Probekissen zugeführt wird.

Die Vorkammer hat daher in etwa das Volumen der beabsichtigten aufzubringenden Probeflüssigkeit. Bei Speichel eines Menschen als Probeflüssigkeit ist dieses Volumen vorzugsweise 0,6 ml bis 8 ml, insbesondere bevorzugt 0,6 ml bis 4 ml.

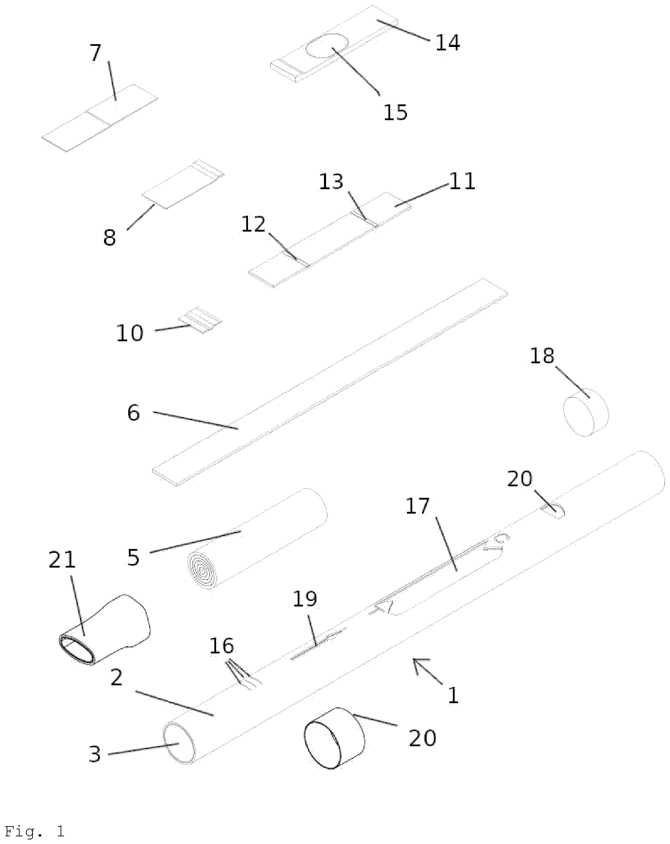

Die erfindungsgemäße Vorrichtung weist ein Gehäuse auf. Das Gehäuse hat eine Probeneintrittsöffnung. Die Probeneintrittsöffnung ermöglicht eine rasche Aufnahme einer Probe in die Vorkammer. Vorzugsweise hat die Probeneintrittsöffnung eine Fläche von 5 mm² bis 50 mm², vorzugsweise von 10 mm² bis 40 mm², insbesondere bevorzugt 15 mm² bis 35 mm². Die Probeneintrittsöffnung hat vorzugsweise eine mundgerechte Form, sodass ein Benutzer direkt eine Probe aus dem Mund des Benutzers in die Vorrichtung, insbesondere die Vorkammer, befördern kann. Die Probeneintrittsöffnung kann teilweise oder gänzlich rund, z.B. oval, teilweise oder gänzlich kreisrund oder teilweise oder gänzlich elliptisch sein oder eine Kombination aus oval, kreisrund und elliptisch. Eine weitere Form ist eine Kombination aus Halbkreisen und teilweisen Ellipsen, die an ihrem Ende, optional an einem oder mehreren Eckpunkten, zusammenstoßen. Eine bevorzugte Form ergibt sich durch Quetschen eines runden Gehäuses, z.B. wie dies in Fig. 1 für das ovale Mundstück (21) gezeigt ist. Durch diese Formen kann die Probeneintrittsöffnung in eine mundgerechte Form gebracht werden. Damit können die Lippen eines Benutzers besser, d.h. mit keiner oder geringer Lippenspannung, die Probeneintrittsöffnung durch den Mund des Benutzers umschlossen werden. Dadurch kann das Risiko eines unerwünschten seitlichen Ausflusses einer Probenflüssigkeit, z.B. Gurgulat, verringert oder verhindert werden.

Die Probeneintrittsöffnung führt zur Vorkammer, z.B. durch eine an die Probeneintrittsöffnung angrenzende Vorkammer. Die Vorkammer soll rasch eine Probenflüssigkeit aufnehmen können und daher etwa das Volumen haben, welches für eine bestimmte Anwendung bzw. ein Probenflüssigkeitsvolumen geeignet ist. Das Volumen kann z.B. 0,6 ml bis 8 ml, insbesondere bevorzugt 0,6 ml bis 4 ml, sein. Die Vorkammer ist räumlich begrenzt, sodass eine Probenflüssigkeit nicht unbeabsichtigt verloren geht. Eine Begrenzung kann durch das Gehäuse der Vorrichtung erfolgen. Die Vorkammer hat vorzugsweise nur die gewünschten Öffnungen, insbesondere zur Probeneintrittsöffnung, zum Probekissen und gegebenenfalls zu der mindestens einen Lüftungsöffnung. Die Vorkammer ist vorzugsweise teilweise oder gänzlich zylinder-, prismen- oder kegelförmig, z.B. ausgehend von einer Grundfläche wie zur Probeneintrittsöffnung beschrieben.

Mit der Vorkammer ist ein Probekissen verbunden, räumlich angrenzend. Dadurch wird rasch Flüssigkeit von der Vorkammer auf das Probekissen geleitet. Das Probekissen sollte rasch die gewünschte Menge Flüssigkeit von der Vorkammer aufnehmen, möglichst einen Großteil der zu untersuchenden Probeflüssigkeit, z.B. mindestens 80% (Volumen-%) davon. Der Übergang von der Vorkammer zum Probekissen kann eine Fläche von 5 mm² bis 50 mm², vorzugsweise von 10 mm² bis 40 mm², insbesondere bevorzugt 15 mm² bis 35 mm² aufweisen.

"Verbunden" oder "In Verbindung steht" bedeutet eine fluidische Verbindung. Eine flüssige Probe kann durch die Teile, insbesondere Kissen, wandern, insbesondere chromatographisch, z.B. durch Sogwirkung. Dazu können die Teile, insbesondere die Kissen, in Berührung stehen, entweder direkt oder unmittelbar oder indirekt mit einem fluidleitenden Material, wie z.B. einem Filter oder dem Übergangskissen (7), welches optional zwischen dem Probekissen (5) und dem Konjugatkissen (8) eingebunden sein kann. Die unterschiedlichen Kissen können aus separaten Bauteilen gefertigt sein oder auch zusammen gefertigt sein, sodass die genannten Bauteile, insb. die Kissen, unterschiedliche Bereiche des in einem gefertigten Materials sein können.

Die Kissen (aus dem engl. "pad") sind für chromatographische Schnelltests ("Lateral-Flow-Tests") bekannte Teile oder Bereiche, wie in der einleitenden Literatur offenbart. Sie können Flüssigkeit aufnehmen und weiterleiten. Sind üblicherweise aus einem Vliesmaterial, wie Glasfaser, oder Nitrocellulose. Nitrocellulose wird insbesondere bevorzugt als Material des Laufstreifens verwendet.

In der erfindungsgemäßen Vorrichtung wird ein Konjugatkissen vorgesehen, welches mit dem Probekissen in Verbindung steht. Diese Verbindung kann eine Berührung der Teile direkt oder über weiterleitende Teile, wie z.B. ein optionales Übergangskissen (engl. wicking pad) erfolgen. Im Konjugatkissen werde in einem chromatographischen Schnelltest (Lateral-Flow-Test) Reagenzien, wie Bindungsreagenzien, und Kontrollreagenzien für den Test vorgesehen, welche durch ein Laufmittel (wie eine Probeflüssigkeit), ggf. nach Analytenbindung, fluidisiert und über den Laufstreifen befördert werden können. In anderen Ausführungsformen ist das Konjugatkissen optional. In diesem Fall kann das Probekissen mit dem Laufstreifen in Verbindung stehen. Reagenzien, die für das Konjugatkissens beschrieben sind, können dann im Probekissen oder im Laufstreifen enthalten sein. Das Konjugatkissen kann optional ein Teil des Probekissens oder des Laufstreifens sein oder mit diesem aus einem Stück gefertigt sein. Bevorzugt ist ein eigener Teil oder Streifen als Konjugatkissen zur einfacheren Bearbeitung und Reagenzienbehandlung in der Herstellung der erfindungsgemäßen Vorrichtung vorgesehen.

Reagenzien, die im Konjugatkissen vorgesehen werden können sind z.B. Bindungspartner eines oder mehrerer Analyten, wie z.B. Antikörper, die einen Analyten binden können, oder Antigene, welche Antikörper, die auch Analyten sein können, binden können, oder andere Bindungspartner von Analyten. Sämtliche Reagenzien, Analyten und Bindungspartner, die für Immunoassays, chromatographischer Tests oder Lateral-Flow-Tests bekannt sind, können erfindungsgemäße eingesetzt werden; bzw. kann die Erfindung zur Detektion sämtlicher Analyten, wie Krankheitserreger oder deren Teile oder Substanzen, bekannter Immunoassays, chromatographischer Tests oder Lateral-Flow-Tests eingesetzt werden. Ist der Analyt ein Antikörper, wird die Vorrichtung auch als Antikörper-Schnelltest bezeichnet. In diesem Fall ist der Bindungspartner vorzugsweise ein diesen Antikörper bindendes Antigen. Ist der Analyt ein Antigen, wird die Vorrichtung auch Antigen-Schnelltest bezeichnet. In diesem Fall ist der Bindungspartner vorzugsweise ein dieses Antigen bindender Antikörper. Die Bindungspartner im Allgemeinen können markiert sein. Die Markierung sollte mit einem detektierbaren Mittel sein, z.B. ein Farbstoff, ein fluoreszierendes Mittel, ein Nanopartikel, z.B. Goldnanopartikel. Denkbar ist auch ein radioaktiver Stoff. Radioaktive Markierungen sind mit hoher Sensitivität detektierbar, sind jedoch aufgrund der aufwendigeren Bestimmung bei Schnelltests weniger bevorzugt. Bevorzugt sind mit freiem Auge erkennbare Mittel, sofern ein positiver Test oder eine positive Kontrolle erfolgt. Die Reagenzien können bei der Herstellung der erfindungsgemäßen Vorrichtung als Flüssigkeit aufgebracht werden und dann eingetrocknet werden. Diese Bindungspartner werden im Konjugatkissen lösbar vorgesehen. Damit können sie chromatographisch zusammen mit der Probeflüssigkeit/den Analyten bewegt werden, insbesondere über den Laufstreifen gemäß dem chromatographischen Prinzip (Sogwirkung).

Weiters wird ein Laufstreifen, welcher mit dem Konjugatkissen in Verbindung steht, vorgesehen. Auch diese Verbindung kann direkt oder indirekt über ein oder mehrere Zwischenelemente sein. Am Laufstreifen werden die fluidisierten Substanzen, Analyten und Kontrollreagenzien, fortbewegt, sodass letztlich eine Trennung und ggf. Reinigung von Substanzen bei zeitweise kontinuierlichem Lauf eines Laufmittels durch den Laustreifen möglich ist. Auf dem Laufstreifen können immobilisierte Bindungsmittel sein, um Analyten oder Kontrollsubstanzen an diskreten Stellen, wie einem oder mehreren Testbereichen und optional einem oder mehreren Kontrollbereichen, zu binden. Durch diese Bindung können sich die Analyten oder Kontrollsubstanzen an diesen diskreten Stellen ansammeln oder aufkonzentrieren, wodurch eine Detektion oder Sichtbarmachung ermöglicht oder verstärkt wird. Die Trennung kann durch das Binden an die Bindungsmittel erfolgen.

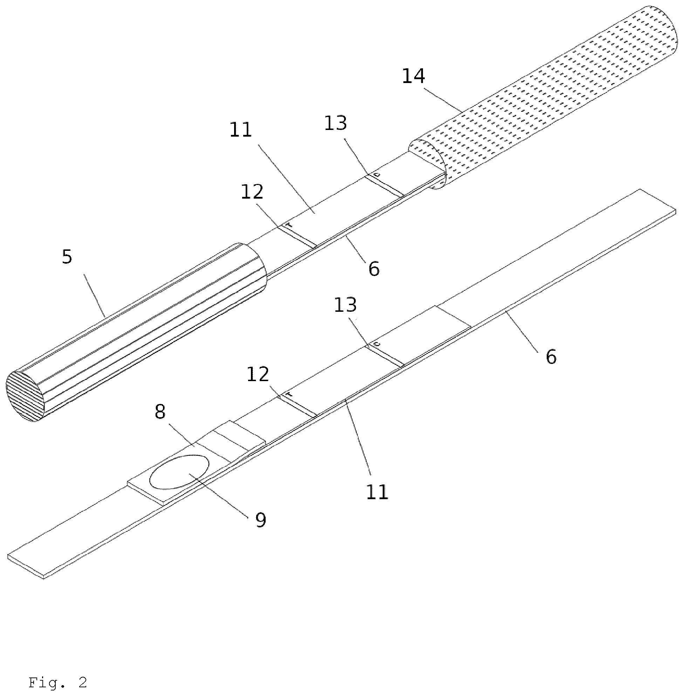

Die Verbindung mit dem Konjugatkissen soll an einem Ende des Laufstreifens sein. An einem weiteren Ende des Laufstreifens ist der Laufstreifen mit einem Saugkissen verbunden. Mit Ende und weiterem Ende des Laufstreifens sind die Bereiche des Laufstreifens gemeint, die sich gegenseitig gegenüberstehen, mit dem Laufbereich, in dem ein Laufmittel vom Konjugatkissen zum Saugkissen läuft, zwischen diesen beiden Enden. In diesem zwischenliegenden Laufbereich befinden sich ein oder mehrere Testbereiche und optional ein oder mehrere Kontrollbereiche. Die Enden beziehen sich somit auf eine relative Positionierung. Sie können, müssen aber nicht, an einer Endkante des Laufstreifens sein. Üblicherweise ist der Kontakt mit dem Konjugatkissen an einem (in Aufsicht) überlappenden Bereich (siehe z.B. Fig. 2, unten). Ebenso kann der Laufstreifen mit dem Saugkissen in einem (in Aufsicht) überlappenden Bereich in Verbindung stehen.

Am Laufstreifen können ein oder mehrere Testbereiche sein. In einem Testbereich werden immobilisierte Bindungsmittel vorgesehen, um den Analyten am Testbereich zu binden. Diese Bindungsmittel können den Analyten direkt binden, oder indirekt, wie z.B. einen Bindungspartner des Analyten. Ein solcher Bindungspartner des Analyten kann im Probekissen oder im Konjugatkissen vorgesehen werden. Dieser Bindungspartner des Analyten wandert zusammen mit dem Analyten durch den Laufstreifen, sind also am Probekissen oder Konjugatkissen nicht immobilisiert, sondern löslich vorgesehen.

Beispielsweise können immobilisierte Bindungsmittel sekundäre Antikörper sein, welche Antikörper, die einen Analyten binden (primäre Antikörper), binden. Die primären Antikörper sind in diesem Beispiel Bindungspartner des Analyten.

Am Laufstreifen können ein oder mehrere Kontrollbereiche sein. Kontrollbereiche können gleich wie die Testbereiche gestaltet und gehandhabt werden, mit dem Unterscheid, dass nicht ein Analyt, sondern ein Kontrollanalyt gebunden wird, der in der Vorrichtung selbst vorgesehen wird, z.B. im Konjugatkissen oder im Probekissen.

In einem Kontrollbereich werden immobilisierte Kontrollanalyt-bindende Mittel vorgesehen, um den Analyten am Testbereich zu binden. Diese Bindungsmittel können den Kontrollanalyten binden. In bevorzugten Ausführungsformen der vorliegenden Erfindung hat der Laufstreifen einen Kontrollbereich mit einem immobilisierten Kontrollanalyt-bindenden Mittel. Vorzugsweise ist das Kontrollanalyt-bindende Mittel ein Kontrollanalyt-bindender Antikörper. Der Kontrollanalyt kann wie oben zum Bindungspartner des Analyten beschrieben markiert sein. Die Markierung kann die gleiche sein, womit ein guter Vergleich zwischen Analytbindung und Kontrollanalytbindung möglich ist. Unterschieden werden können die beiden Bindungen durch die unterschiedliche räumliche Platzierung des Testbereichs und Kontrollbereichs am Laufstreifen. Bei Markierung mit Farbstoffen können auch unterschiedliche Farbstoffe mit unterschiedlichen Farben gewählt werden, um die Unterscheidungskraft noch zu erhöhen. Bevorzugt werden Nanopartikel, z.B. Goldnanopartikel.

Vorzugsweise enthält das Probekissen oder Konjugatkissen einen Kontrollanalyten, welcher bei Fluidisierung mit einem Laufmittel am Laufstreifen befördert werden kann. Der Kontrollanalyt wandert zusammen mit dem Analyten durch den Laufstreifen, ist also am Konjugatkissen oder Probekissen nicht immobilisiert, sondern löslich vorgesehen.

Die erfindungsgemäße Vorrichtung weist weiters ein Saugkissen auf, welches mit dem Laufstreifen an einem weiteren Ende des Laufstreifens in Verbindung steht. Das Saugkissen ist vorzugsweise aus einem Material, welches durch Saugwirkung Flüssigkeit vom Laufstreifen aufnehmen kann. Es ist vorzugsweise aus einem Filz- oder Vliesmaterial, z.B. ein Glasfaservlies. Die Verbindung mit dem Laufstreifens ist vorzugsweise direkt, z.B. durch Kontakt, wie an einem Überlappungsbereich. Das Saugkissen ist der letzte Bereich, den eine Probenflüssigkeit beim Fluss durch die erfindungsgemäße Vorrichtung in sachgemäßer Benutzung erreicht. Im Probekissen sammelt sich somit die Probenflüssigkeit, nachdem sich Analyten und Kontrollanalyten bereits am Laufstreifen in den vorgesehen Bereichen angesammelt haben (sofern der Analyt in der Probe vorhanden war). Abgesehen von der Flüssigkeit, welche in den zuvorliegenden Kissen und Streifen zurückbleibt, wird ein Teil der Probenflüssigkeit zum Saugkissen befördert. Die zum Saugkissen beförderte Probenflüssigkeitsmenge sollte ausreichen, um bei dem ausgewählten Textsystem (zu erwartende Analytmenge und Probenart) ausreichend Analyt (sofern in positiver Probe vorhanden) durch den Laufstreifen zu befördern. Z.B. kann das Saugkissen ein Volumen von mindestens 10% vorzugsweise mindestens 20%, mehr bevorzugt mindestens 40%, besonders bevorzugt mindestens 60%, speziell bevorzugt mindestens 80%, des Volumens des Probekissens aufweisen (alle % in Volumen-ö).

Erfindungsgemäß hat das Gehäuse im Bereich der Vorkammer, vorzugsweise von der Probeneintrittsöffnung aus gesehen der Länge nach in ihrem letzten Drittel vor dem Probekissen, eine Lüftungsöffnung. Die Lüftungsöffnung in der Vorkammer, knapp vor dem Probekissen, kann eine Entlüftung der Vorkammer bewirken, sodass Luftblasenbildung oder Luftblasen, welche die Vorkammer blockieren könnten oder den Probenfluss zum Probekissen hindern können, vermieden bzw. entfernt werden. Es sind auch mehrere Lüftungsöffnungen, z.B. 2, 3, 4 oder mehr, möglich. Eine Lüftungsöffnung ist vorzugsweise klein, sodass Luft entweichen kann aber Probenflüssigkeit weitgehend zurückgehalten wird, z.B. aufgrund der Oberflächenspannung von Wasser und/oder der raschen Sogwirkung des Probekissens auf die Probenflüssigkeit. Besonders bevorzugt ist eine Lüftungsöffnung in Spaltform, z.B. mit 1 mm bis 5 mm Länge und 0,5 mm oder weniger Breite. Die Lüftungsöffnung ist ein Durchbruch im Gehäuse und verbindet die Vorkammer mit der Umgebung.

Vorzugsweise kann auch eine oder mehrere Lüftungsöffnung(en) im Bereich des Probekissens vorgesehen werden. Insbesondere kann somit das Gehäuse im Bereich des Probekissens, vorzugsweise von der Probeneintrittsöffnung aus gesehen der Länge nach in seinem ersten Viertel nach der Vorkammer, eine weitere Lüftungsöffnung haben. Die Dimensionen dieser weiteren Lüftungsöffnung(en) sind vorzugsweise wie zuvor beschrieben. Die Lüftungsöffnung ist ein Durchbruch im Gehäuse und/oder eine Ausnehmung davon und verbinden den Raum des Probekissens mit der Umgebung. Da das Probekissen luftdurchlässig ist, kann auch im Bereich des Probekissens eine Entlüftung durch diese weitere Lüftungsöffnung erfolgen. Die zuvor zur Lüftungsöffnung im Bereich der Vorkammer beschriebenen Vorteile kommen auch hier zu tragen. Weiters kann auch eine Entlüftung erfolgen, wenn die Lüftungsöffnung im Bereich der Vorkammer verstopft wird, z.B. durch Schleim oder zähen Speichel, der mit der Probeflüssigkeit in den Vorraum eingetragen werden kann. Schleim oder zäher Speichel kann z.B. bei einer Erkrankung des Benutzers auftreten. Da das Probekissen derartigen Schleim zurückhalten kann, kann mit der Lüftungsöffnung im Bereich des Probekissen weiterhin eine Entlüftung und damit Entfernung von Luftblasen aus der Vorkammer über den Raum des Probekissens erfolgen.

In den bevorzugten Ausführungsformen ummantelt das Gehäuse die Vorkammer, das Probekissen, das Konjugatkissen, den Laufstreifen und das Saugkissen, jeweils zumindest teilweise. Durch die Ummantelung, insbesondere im Bereich der Vorkammer und des Probekissens, wird Probeflüssigkeit zum Teststreifen (Laufstreifen) geleitet ohne an die Umgebung wesentlich verloren zu gehen. Die Ummantelung von Bereichen mit dem Konjugatkissen, den Laufstreifen und dem Saugkissen ist optional und in manchen Ausführungsformen nur teilweise (Sichtfenster) und hat hauptsächlich den Zweck einer Halterung dieser Kissen und Streifen. Die Vorkammer ist vorzugsweise weitgehend durch das Gehäuse ummantelt (natürlich abgesehen von der hierin beschriebenen einen oder mehreren Lüftungsöffnungen). Die Vorkammer kann durch einen Teil des Gehäuses ausgebildet sein. Vorzugsweise sind von der Probeneintrittsöffnung aus gesehen die Vorkammer, das Probekissen, das Konjugatkissen, der Laufstreifen und das Saugkissen gemäß der genannten Reihenfolge innerhalb des Gehäuses angeordnet. Diese Reihenfolge entspricht der Flussrichtung einer Probenflüssigkeit durch den Lateral-Flow-Test.

Vorzugsweise hat das Gehäuse im Bereich des Laufstreifens ein Sichtfenster. Das Sichtfenster ist ein Durchbruch im Gehäuse und ermöglicht Sicht von der Umgebung insbesondere zu den Testbereich(en) und Kontrollbereich(en). Die Testbereich(e) und Kontrollbereich(e) können damit von einem Benutzer abgelesen werden.

Gemäß der Erfindung kann in der Vorrichtung ein Amylasesubstrat in fluidischer Verbindung mit dem Saugkissen verbunden sein. Dazu kann das Amylasesubstrat direkt auf das Saugkissen aufgetragen sein. Insbesondere kann das Amylasesubstrat in einem Indikatorbereich am Saugkissen aufgebracht sein. Der Indikatorbereich bzw. das Amylasesubstrat darin kann durch ein Indikatorfenster im Gehäuse von außen sichtbar sein - in Analogie zum Sichtfenster des Laufstreifens. Das Saugkissen und damit das Amylasesubstrat sind die letzten Bereiche, die eine Probenflüssigkeit beim Fluss durch die erfindungsgemäße Vorrichtung in sachgemäßer Benutzung erreicht. Im Probekissen sammelt sich somit die Probenflüssigkeit. Die Probeflüssigkeit kann Amylase enthalten, welche das Amylasesubstrat abbaut. Diese Abbaureaktion kann mit einem Indikator beobachtet werden, z.B. einem Farbumschlag. Die Abbaureaktion kann benutzt werden um 1) abzuschätzen, ob ausreichend Zeit für die Durchführung eines Tests eingeräumt wurde, und/oder 2) abzuschätzen, ob ausreichend Probe oder Probeflüssigkeit für einen Test eingesetzt wurde. Die Menge an Amylasesubstrat wird daher mit der vorgesehenen Probeflüssigkeitsmenge abgestimmt, z.B. indem bei der Herstellung durch einfache kontrollierte Tests die Abbauzeit bzw. ein Indikatorumschlag beobachtet wird und die Amylasesubstratmenge daraufhin mit der genwünschten Zeit bzw. Probeflüssigkeitsmenge abgestimmt wird.

Amylase und ihre Substrate sind bekannt. Ein bevorzugtes Amylasesubstrat umfasst oder ist ein Amylose-Iod-Konjugat, vorzugsweise ein Stärke-Iod-Konjugat. Stärke-Iod-Konjugat ist auch als Lugolsche Mischung oder Lugolsche Lösung bekannt. Iod kann als elementares Iod oder eines Iodidsalzes oder einer Mischung davon vorgesehen werden. Beispielsweise können 0,01 µg bis 10 µg Stärke und 50 µg bis 1000 µg Iod vorgesehen werden. In einer anderen Ausführungsform wird eine Mischung aus 0,01 µg bis 10 µg Stärke und 50 µg bis 500 µg Iod und 100 µg bis 800 µg Kaliumiodid vorgesehen. Die Iod-Stärke Reaktion wird auch als Iodprobe bezeichnet und führt zu einer blauen Färbung. Durch den Abbau der Amylase bzw. Stärke verblasst die blaue Farbe.

Bei der erfindungsgemäßen Vorrichtung kann an zumindest einer der Lüftungsöffnungen (16) ein Feuchtigkeitsindikator angebracht sein. Beispielsweise kann der Feuchtigkeitsindikator an einem luftdurchlässigen Indikatorträger angebracht sein, z.B. ein poröser Indikatorträger, wie ein Vlies, auf welchem der Feuchtigkeitsindikator angebracht ist. Der Indikatorträger kann an der zumindest einen Lüftungsöffnungen (16) anliegen. Vorzugsweise ist der Feuchtigkeitsindikator bzw. der Indikatorträger an einer in Fließrichtung hintersten oder letzten Lüftungsöffnung angebracht, z.B. an einer, die im Bereich des Probekissens ist. Der Indikatorträger soll nach Möglichkeit luftdurchlässig sein, sodass eine Lüftungsfunktion der abgedeckten Lüftungsöffnung weiterhin besteht. Der Indikatorträger kann beispielsweise badförmig sein und/oder das Gehäuse umfassen, z.B. wie ein Ring. Der Indikatorträger kann ein Band, Etikett, Pflaster oder Banderole sein. Feuchtigkeitsindikatoren sind hinlänglich bekannt, wie z.B. Cobaltdichlorid oder Methylviolett, welche einen Farbumschlag beim Übergang vom trockenen zum feuchten Zustand zeigen. Eine andere Möglichkeit für einen Feuchtigkeitsindikator ist ein pH-Indikator, sofern mit einem feuchten Zustand auch ein pH Umschlag verbunden ist. Dafür sollte der Feuchtigkeitsindikator im trockenen Zustand bei einem pH vorliegen als ein Gurgulat eines Menschen, sodass ein Farbumschlag des pH Indikators beobachtet werden kann, sobald er mit einer Probe in Kontakt kommt. Generell sind Feuchtigkeitsindikatoren, welche einen Farbumschlag beim Übergang vom trockenen in den feuchten Zustand anzeigen, bevorzugt. Die erfindungsgemäße Vorrichtung wird mit trockenem Feuchtigkeitsindikator geliefert. Wenn bei Gebrauch der Flüssigkeit an den Feuchtigkeitsindikator gelangt, wird dies angezeigt. Der Feuchtigkeitsindikator wirkt als Füllstandsanzeige, d.h. wenn bei Gebrauch die Vorkammer vollläuft und Flüssigkeit durch die Lüftungsöffnung tritt, so wird dies am Feuchtigkeitsindikator angezeigt und der Benutzer sieht, dass keine weitere Probenflüssigkeit eingebracht werden soll. Der Indikatorträger kann auch eine Aufnahmefunktion haben und austretende Probenflüssigkeit aufnehmen, sodass diese nicht entlang des Gehäuses rinnt und eventuell in den Messbereich (Laufsteifen) gelangt, ohne zuvor die Probe- und Konjugatkissen passiert zu haben. Ein Benutzer kann eine derartige Kontamination auch mit entsprechender Fingerplatzierung am Gehäuse verhindern. Ein Indikatorträger, welcher austretende Probeflüssigkeit aufnehmen kann, hat den Vorteil, dass dies nicht erforderlich ist.

Die Erfindung betrifft auch die Verwendung der Vorrichtung zur Bestimmung eines Analyten. Insbesondere betrifft die Erfindung ein Verfahren zur Bestimmung eines Analyten mit einer Vorrichtung gemäß der Erfindung, umfassend Aufbringen einer flüssigen Probe (Probenflüssigkeit) in die Vorkammer der Vorrichtung, wobei die flüssige Probe durch das Probekissen aufgesogen wird und durch weitere Sogwirkungen des damit verbundenen Konjugatkissens, des damit verbundenen Laufstreifens und des damit verbundenen Saugkissens zumindest teilweise durch das Konjugatkissen, durch den Laufstreifen, zum Saugkissen befördert wird, wobei in einem Testbereich am Laufstreifen der Analyt gebunden wird. Ein Teil der Probe wird in den jeweiligen Elementen (Kissen, Streifen) verbleiben, weswegen die Probe nur zumindest teilweise durch die Elemente befördert wird oder nur teilweise das Saugkissen erreicht. Die Funktion bzw. der Probenfluss ist wie oben zu den einzelnen Elementen beschrieben.

Vorzugsweise wird der Analyt mit der flüssigen Probe (Probenflüssigkeit) aufgebracht. Alternativ wird der Analyt durch eine Reaktion im Probekissen und/oder im Konjugatkissen oder in einem weiteren Kissen welches fluidisch dem Laufstreifen vorgereiht ist, aus einer Komponente der flüssigen Probe gebildet werden. So kann beispielsweise ein Analytvorläufer (der in der flüssigen Probe sein kann) durch eine Reaktion in den Analyten umgewandelt werden. Mögliche Reaktionen eines Analytvorläufers zu einem Analyten sind Oxidation, Reduktion, enzymatische Umwandlung, insbesondere enzymatische Spaltung, Komplexbildungsreaktion, Säure-Base Reaktion, etc.. Der Analyt kann auch aus einer Matrix der Probe herausgeöst werden, z.B. durch ein Detergens. Reagenzien für eine derartige Reaktion werden vorzugsweise im Probekissen vorgelegt, um bei Kontakt mit der Probe die Reaktion zu bewirken.

Besonders bevorzugt ist der Analyt ein Bestandteil eines Pathogens, wie ein Virus, Bakterium oder Pilz. Mögliche Bestandteile sind Oberflächenmoleküle des Pathogens, wie z.B. ein Membranprotein oder ein Kapsidprotein, ein Glykoprotein, ein Peptidoglykan, oder ein anderes beliebiges Protein oder Molekül des Pathogens, welches ggf. freigesetzt werden kann. Eine Freisetzung eines Analyten kann z.B. durch eine enzymatische Reaktion, z.B. Abspalten eines Proteinbestandteils, oder durch Entfernung der Membran des Pathogens erfolgen, z.B. durch ein Detergens, ein Tensid oder einen Emulgator oder Kombinationen davon bzw. Substanzen die sowohl Detergens-, Tensid- und/oder Emulgatorwirkung haben. Bevorzugte Detergenzien und Emulgatoren sind ionische Tenside, nicht-ionische Tenside, ionische Detergenzien, nicht-ionische Detergenzien. Beispiele sind ein Polysorbat (z.B. Polysorbat 20, "Tween-20"), Polyethylenglykol p-(1,1,3,3-tetramethylbutyl)-phenylether ("Triton X-100"), ein Phenoxypolyethoxylethanol, z.B. Nonylphenoxypolyethoxylethanol ("NP-40" oder "Tergitol"). Weiters kann ein oder mehrere Chelatbildner vorgesehen werden, z.B. Zitronensäure oder EDTA, welche Ionen für enzymatische Reaktionen maskieren können, also die Verfügbarkeit der Ionen für die Enzyme reduzieren. Die Chelatbildner werden ebenfalls bevorzugt im Probekissen vorgelegt. Weiters kann das Probekissen Buffer oder Salze aufweisen.

Die erfindungsgemäße Vorrichtung ist insbesondere zur Verwendung von Speichel oder Sputum als Probeflüssigkeit geeignet. Diese kann direkt vom Mund eines Benutzers in die Vorrichtung, insbesondere in die Vorkammer durch die Probeneintrittsöffnung eingebracht werden. Dazu nimmt der Benutzer die Vorrichtung, konkret das Ende mit der Probeneintrittsöffnung in den Mund, und lässt Probeflüssigkeit vom Mund in die Vorkammer fließen. Vorzugsweise ist für diese Probeneinbringungsmethode die Probeneintrittsöffnung in einer mundgerechten Form, wie oben beschrieben, z.B. oval. Allfällige Über- oder Unterdrücke durch Blasen oder Ansaugen werden - wie oben betont - durch die Lüftungsöffnung minimiert oder vermieden. Alternativ kann die Probe zunächst auf einem Probenabstrichstab aufgebracht werden, z.B. als Rachen- oder Nasenabstrich. Vom Probenabstrichstab kann die Probe mit Flüssigkeit herunter gespült werden. Die abgespülte Probe ist dann die Probenflüssigkeit, welche auf das Probekissen aufgebracht wird. Eine Methode hierzu ist den Probenabstrichstab in die Vorkammer zu bringen und dann innerhalb des Gehäuses die Probe vom Probenabstrichstab herunter zu spülen. Das herunter spülen erfolgt vorzugsweise in senkrechter Position des Gehäuses. Hierzu ist die Probeneintrittsöffnung vorzugsweise rund, z.B. kreisrund. Nach Aufbringen der Probe in die Vorkammer, wandert die Probenflüssigkeit aufgrund von Sogwirkungen der Materialien durch die Elemente der Vorrichtung Richtung Saugkissen, wie oben beschrieben. Der Benutzer muss hierzu nicht aktiv werden und kann abwarten, bis ein Resultat im Testbereich und/oder Kontrollbereich, ggf. auch im Indikatorbereich sichtbar wird. Üblicherweise dauert ein Testablauf 2 min bis 15 min. Durch Abbau des Amylasesubstrats kann der Testabschluss gut von einem Benutzer eingeschätzt werden. Im Kontrollbereich sollte bei positiver Kontrolle stets ein Signal ersichtlich sein. Im Testbereich nur bei positivem Testergebnis, also bei Vorliegen des Analyten (der seines Vorläufers) in der Probenflüssigkeit.

Um genügen Probeflüssigkeit zur Verfügung zu stellen, kann dem Benutzer auch eine Flüssigkeit zur Verfügung gestellt werden, z.B. zum Gurgeln im Fall eines Gurgulats als Probeflüssigkeit ("Gurgelflüssigkeit"). Hierzu kann die erfindungsgemäße Vorrichtung auch als Kit zusammen mit einer Flüssigkeit, z.B. 1 ml bis 20 ml, zur Verfügung gestellt werden. Die Flüssigkeit des Kits ist vorzugswese eine wässrige Salzlösung, z.B. eine physiologische Salzlösung, insbesondre bevorzugt 9 g/l Kochsalz oder Ringerlösung. Die Flüssigkeit ist bevorzugt eine Gurgelflüssigkeit. Die Flüssigkeit kann in einem Behälter, z.B. einer Flasche, im Kit zur Verfügung gestellt werden. Ebenso kann die Vorrichtung mit einem Probenabstrichstab zur Verfügung gestellt werden. Mit dem Probenabstrichstab kann ein Abstrich von einer Person wie oben beschrieben genommen werden. Vom Stab kann die Probe herunter gespült werden, z.B. mit einer Spülflüssigkeit. Spülflüssigkeit kann ebenfalls eine wässrige Salzlösung, wie oben, sein. Die Erfindung betrifft somit auch einen Kit mit einer erfindungsgemäßen Vorrichtung und einem Probenabstrichstab, und/oder mit einem Behälter mit einer Spülflüssigkeit oder Gurgelflüssigkeit.

Ebenfalls liefert die Erfindung ein Herstellungsverfahren zur Produktion einer Vorrichtung gemäß der Erfindung, umfassend Einbringen des Probekissens, des Konjugatkissens, des Laufstreifens und des Saugkissens in das Gehäuse mit der Probeneintrittsöffnung und der mindestens einen Lüftungsöffnung, wobei das Gehäuse im Bereich der Vorkammer mindestens eine Lüftungsöffnung hat. Das Gehäuse wird üblicherweise zuvor gefertigt, z.B. als Rohr. Die anderen Komponenten (Kissen, Streifen) werden auf einen Träger aufgelegt und ggf. angeklebt. Der Träger wird dann mit den Kissen und Streifen in das Gehäuse eingeschoben.

Zwecks einer kostengünstigen Produktion ist es vorteilhaft, wenn das Gehäuses aus einem Pappkarton besteht. Die Verwendung von Pappkarton hat außerdem einen positiven umwelttechnischen und funktionellen Aspekt. Der Pappkarton kann flüssigkeitsresistent sein oder innen eine flüssigkeitsresistente Schicht aufweisen.

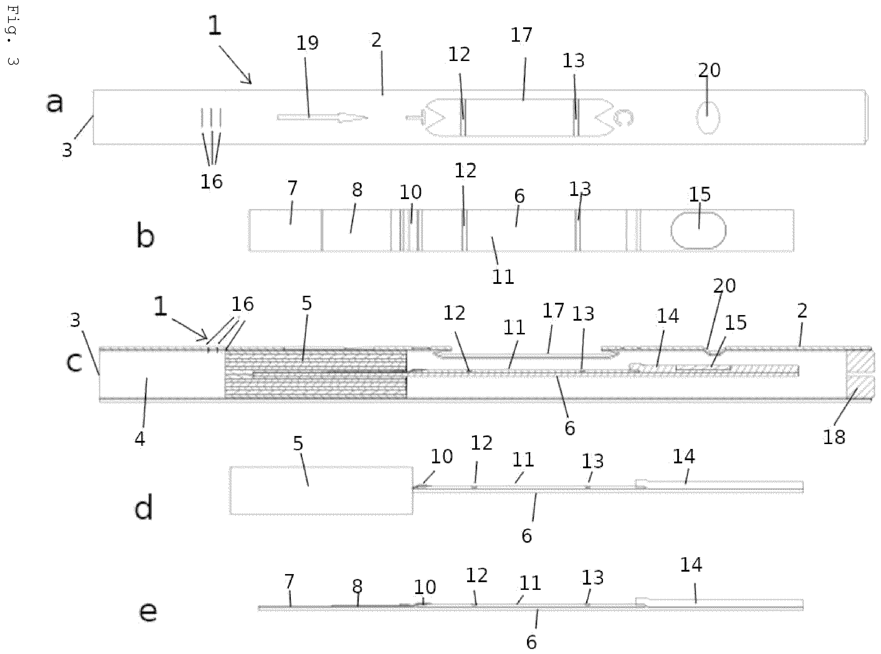

Unter Bezugnahme auf die Figuren wird eine besonders bevorzugte Ausführungsform der erfindungsgemäßen Vorrichtung für einen Lateral-Flow-Test beschrieben. Allgemein können beliebige Elemente davon mit den zuvor beschriebenen Ausführungsformen kombiniert werden. Die erfindungsgemäße Vorrichtung für einen Lateral-Flow-Test (1) hat ein Gehäuse (2), welches weitgehend rohrförmig ist. An einem Ende des Gehäuses (Rohres) ist eine Probeneintrittsöffnung (3), durch die eine Probenflüssigkeit bei Benutzung aufgenommen werden kann, vorgesehen. Die Probeneintrittsöffnung führt in die Vorkammer (4), worin Probenflüssigkeit für kurze Zeit bei Benutzung aufgehalten werden kann, bis die Probenflüssigkeit durch das Probekissen (5), welches mit der Vorkammer in Verbindung steht, aufgenommen wird. Im Bereich der Vorkammer werden, wie in Fig. 3c gezeigt, zwei Lüftungsöffnungen (16) und im Bereich des Probekissens eine Lüftungsöffnung (16) vorgesehen. Die Probenflüssigkeit gelangt vom Probekissen über ein Übergangskissen (7), welches den Probeflüssigkeitsdurchfluss einstellt, auf ein Konjugatkissen (8). Am Konjugatkissen können durchgehend oder in einem konkreten Konjugatbereich (9) Bindungspartner zur Bindung des Analyten und Kontrollanalyten vorgesehen werden. Diese lösen sich und wandern mit der Probeflüssigkeit.

In Verbindung mit dem Konjugatkissen ist ein Laufstreifen (11) vorgesehen. Zwischen Laufstreifen oder dem Laufstreifenseitigem Ende des Konjugatstreifens und dem Probekissen kann eine Abdeckschicht (10) vorgesehen werden, welche eine Trennung des Laufstreifens oder des Konjugatstreifens vom Probekissen bewirkt, sodass keine Probeflüssigkeit vom Probekissen direkt auf den Laufstreifen gelangt und damit gewährleistet wird, dass die Probeflüssigkeit bevor sie auf den Laufstreifen gelangt zunächst das Konjugatkissen (bzw. den Konjugatbereich) und die Reagenzien darauf durchwandert.

Am Laufstreifen wird ein Testbereich (12) und ein Kontrollbereich (13) vorgesehen, an denen Antikörper, welche Analyten bzw. Kontrollanalyten binden, immobilisiert sind. Der Testbereich und der Kontrollbereich sind durch ein Sichtfenster (17) aus der Umgebung und durch einen Benutzer sichtbar. Das Sichtfenster ist eine Ausnehmung oder Öffnung im Gehäuse. Der Testbereich ist in Fig. 1, 2 und 3a mit einem "T" für "Test" markiert; der Kontrollbereich mit einem "C" für "control" (Kontrolle).

Am anderen Ende des Laufstreifens ist dieser mit dem Saugkissen (14) in Kontakt. Das Saugkissen kann wie in Fig. 1 und 3 dargestellt als Streifen ausgeführt werden oder wie in Fig. 2 dargestellt als voluminöses Vlies, welches optional das Gehäuse ausfüllen kann. Auf dem Saugkissen ist in der Ausführungsform der Fig. 1 ein Indikatorbereich (15) vorgesehen, auf dem eine Lugolsche Lösung aufgebracht und getrocknet wird. Dies dient als Indikator für Speichel. Die Amylase im Speichel kann die Stärke der Lugolschen Lösung zersetzen und eine Farbveränderung von blau zu farblos bewirken, üblicherweise innerhalb von etwa 10 min nach Kontakt mit der Amylase-haltigen Probeflüssigkeit. Der Indikatorbereich ist durch ein Indikatorfenster (20) aus der Umgebung und damit durch einen Benutzer sichtbar. Das Indikatorfenster ist eine Ausnehmung im Gehäuse.

Der Laufstreifen und der Konjugatstreifen, ggf. (gegebenenfalls) auch das Saugkissen werden auf einem Träger (6) aufgelegt. Die Streifen können auch aufgeklebt sein. Der Träger ist üblicherweise aus einem Probeflüssigkeits-undurchlässigen (wasserundurchlässigen) Material, z.B. Kunststoff.

Am der Probeneintrittsöffnung gegenüberliegenden Ende des Gehäuses kann das Gehäuse mit einem Verschluss (18) verschlossen sein. Im Grunde ist dies für die Funktion nicht erforderlich, jedoch fördert er die Handhabung, da ein Benutzer davon abgehalten wird irrtümlich eine Probe an diesem Ende in das Gehäuse einzubringen. Zur Veranschaulichung der Funktion kann am Gehäuse ein Fließrichtungsanzeiger (19) angebracht werden, in Fig. 3a ist dies ein Pfeil, der die Fließrichtung von der Probeneintrittsöffnung in Richtung Laufstreifen anzeigt.

In einer konkreten Ausführungsform wird die erfindungsgemäße Vorrichtung als Corona-Schnelltest verwendet. Analyt ist hierbei ein Protein eines Coronavirus', z.B. von SARS-CoV-2. Ein vorteilhaftes Protein ist eines, welches selten mutiert wird, um Probleme der Detektion von Mutanten zu vermeiden. Ein solches Protein ist das Nukleokapsidprotein. Um dieses für den Test verfügbar zu machen, werden im Probekissen Detergenzien vorgesehen, insbesondere, die oben genannten. Dadurch wird bei Kontakt des Probekissens mit der Probeflüssigkeit das Detergens gelöst und die Virushülle aufgebrochen oder gelöst und des Nukelkapsid freigesetzt. Das freigesetzte Nukleokapsid wandert mit der Probeflüssigkeit auf das Konjugatkissen, in dem ein Antikörper gegen das Nukelokapsid vorgesehen ist. Der Antikörper ist mit einem Goldnanopartikel markiert. Der Antikörper wird durch die Probeflüssigkeit gelöst und komplexiert durch eine Antigen-Antikörper-Reaktion das Nukleokapsidprotein. Der Komplex gelangt zum Testbereich am Laufstreifen. Dort ist ein immobilisierter weiterer Antikörper vorgesehen, der das Nukleokapsidprotein bindet. Das Nukleokapsidprotein wird also an zwei unterschiedlichen Stellen durch den markierten Antikörper vom Konjugatkissen und den immobilisierten Antikörper gebunden. Durch diese Bindung reichert sich der markierte Antikörper im Testbereich an und eine Färbung durch Anreicherung der Goldpartikel wird sichtbar (positiver Test). Bei einer negativen Probe ohne Analyten kommt es zu keiner Anreicherung im Testbereich; der gelöste markierte Antikörper wird ohne Antigenbindung weiter gespült und gelangt teilweise zum Saugkissen.

Eine ähnliche Reaktion erfolgt im Kontrollbereich, nur ist diese nicht von der Anwesenheit eines Analyten in der Probeflüssigkeit abhängig. Es kann beispielsweise ein markierter Antikörper im Konjugatkissen vorgesehen werden. Dieser wandert zum Kontrollbereich und wird dort von einem sekundären Antikörper, der nur diesen Antikörper (als Kontrollanalyt), und nicht einen analytspezifischen-Antikörper, bindet. Dadurch wird die Markierung (Goldpartikel) am Kontrollbereich sichtbar, wodurch ein Benutzer die Funktion des Testverlaufs beurteilen kann.

Am Indikatorbereich findet die Amylasereaktion mit einer Lugolschen Mischung statt. Der Bereich entfärbt von einem kräftigen blau zu hellblau oder farblos bei Kontakt mit Speichel in etwa 5 bis 10 min. Dies bestätigt, dass tatsächlich Speichel als Probeflüssigkeit im Testverlauf verwendet wurde und ausreichend Zeit vergangen ist, damit der Test als abgeschlossen gelten kann.

In einer weiteren Ausführungsform ist die Probeneintrittsöffnung (3) in Form eines ovalen Mundstücks (22), wie in Fig. 1 gezeigt. Vom ovalen Mundstück erstreckt sich das Gehäuse (2) wie in Fig. 1 für eine kreisförmige Probeneintrittsöffnung gezeigt. Das gesamte Gehäuse ist für das ovalen Mundstücks (22) in Fig. 1 nicht separat gezeigt. Ein ovales Mundstück kann z.B. durch Quetschen des Gehäuses im Bereich der Probeneintrittsöffnung entstehen.

In einer bevorzugten Ausführungsform wird über einer Lüftungsöffnung (16) ein Band (21) als Indikatorträger für einen Feuchtigkeitsindikator vorgesehen. Das Band (21) wird ringförmig um das Gehäuse angebracht und deckt eine oder mehrere Lüftungsöffnungen (16) ab. Das Band ist vorzugsweise aus einem luftdurchlässigen Vlies. Feuchtigkeitsindikatoren werden wie oben beschrieben vorgesehen. Bevorzugt ist das Band (21) um die in Fließrichtung hintersten, d.h. dem Probekissen (5) oder dem Sichtfenster (17) am nächsten befindlichen, Probeneintrittsöffnung (3) angebracht.

### Bezugszeichenliste

- 1: Vorrichtung für einen Lateral-Flow-Test
- 2: Gehäuse
- 3: Probeneintrittsöffnung
- 4: Vorkammer
- 5: Probekissen
- 6: Träger
- 7: Übergangskissen
- 8: Konjugatkissen
- 9: Konjugatbereich
- 10: Abdeckschicht
- 11: Laufstreifen
- 12: Testbereich
- 13: Kontrollbereich
- 14: Saugkissen
- 15: Indikatorbereich
- 16: Lüftungsöffnung
- 17: Sichtfenster
- 18: Verschluss
- 19: Fließrichtungsanzeiger
- 20: Indikatorfenster
- 21: Band
- 22: ovales Mundstück

## Patentansprüche

1. Eine Vorrichtung (1) für einen Lateral-Flow-Test aufweisend:
- ein Gehäuse (2), welches eine Probeneintrittsöffnung (3) hat,
- eine an die Probeneintrittsöffnung (3) angrenzende Vorkammer (4),
- ein mit der Vorkammer (4) verbundenes Probekissen (5),
- ein Konjugatkissen (8), welches mit dem Probekissen (5) in Verbindung steht,
- einen Laufstreifen (11), welcher mit dem Konjugatkissen (8) an einem Ende des Laufstreifens (11) in Verbindung steht,
- ein Saugkissen (14), welches mit dem Laufstreifen (11) an einem weiteren Ende des Laufstreifens (11) in Verbindung steht;
wobei das Saugkissen ein Amylasesubstrat aufweist.

2. Die Vorrichtung nach Anspruch 1, wobei das Gehäuse im Bereich der Vorkammer von der Probeneintrittsöffnung aus gesehen der Länge nach in ihrem letzten Drittel vor dem Probekissen (5) und/oder im Bereich des Probekissens (5) mindestens eine mit der Vorkammer in Verbindung stehende Lüftungsöffnung (16) hat.

3. Eine Vorrichtung (1) für einen Lateral-Flow-Test aufweisend:
- ein Gehäuse (2), welches eine Probeneintrittsöffnung (3) hat,
- eine an die Probeneintrittsöffnung (3) angrenzende Vorkammer (4),
- ein mit der Vorkammer (4) verbundenes Probekissen (5),
- ein Konjugatkissen (8), welches mit dem Probekissen (5) in Verbindung steht,
- einen Laufstreifen (11), welcher mit dem Konjugatkissen (8) an einem Ende des Laufstreifens (11) in Verbindung steht,
- ein Saugkissen (14), welches mit dem Laufstreifen (11) an einem weiteren Ende des Laufstreifens (11) in Verbindung steht;
wobei das Gehäuse im Bereich der Vorkammer von der Probeneintrittsöffnung aus gesehen der Länge nach in ihrem letzten Drittel vor dem Probekissen (5) und/oder im Bereich des Probekissens (5) mindestens eine mit der Vorkammer in Verbindung stehende Lüftungsöffnung (16) hat.

4. Die Vorrichtung nach Anspruch 1, 2 oder 3, wobei das Gehäuse im Bereich der Vorkammer von der Probeneintrittsöffnung aus gesehen der Länge nach in ihrem letzten Drittel vor dem Probekissen (5) eine Lüftungsöffnung (16) hat und im Bereich des Probekissens (5) von der Probeneintrittsöffnung (3) aus gesehen der Länge nach in seinem ersten Viertel nach der Vorkammer (4) eine weitere Lüftungsöffnung (16) hat.

5. Die Vorrichtung nach Anspruch 1, 2, 3 oder 4, wobei das Gehäuse (2) die Vorkammer (4), das Probekissen (5), das Konjugatkissen (8), den Laufstreifen (11) und das Saugkissen (14) jeweils zumindest teilweise ummantelt, vorzugsweise wobei das Gehäuse (2) im Bereich des Laufstreifens (11) ein Sichtfenster (17) hat.

6. Die Vorrichtung nach einem der Ansprüche 1 bis 5, wobei die Vorkammer (4) durch einen Teil des Gehäuses (2) ausgebildet wird.

7. Die Vorrichtung nach einem der Ansprüche 1 bis 6, wobei von der Probeneintrittsöffnung (3) aus gesehen die Vorkammer (4), das Probekissen (5), das Konjugatkissen (8), der Laufstreifen (11) und das Saugkissen (14) gemäß der genannten Reihenfolge innerhalb des Gehäuses (2) angeordnet sind.

8. Die Vorrichtung nach einem der Ansprüche 1 bis 7, wobei das Konjugatkissen (8) oder das Probekissen (5) einen Kontrollanalyten enthält, welcher bei Fluidisierung mit einem Laufmittel am Laufstreifen (11) befördert werden kann, vorzugsweise wobei der Laufstreifen (11) einen Kontrollbereich (9) mit einem immobilisierten Kontrollanalyt-bindenden Mittel hat, vorzugsweise wobei das Kontrollanalyt-bindenden Mittel ein Kontrollanalyt-bindender Antikörper ist.

9. Die Vorrichtung nach einem der Ansprüche 1 bis 8, mit einem Amylasesubstrat in fluidischer Verbindung mit dem Saugkissen (14) .

10. Die Vorrichtung nach Anspruch 1, 2 oder 9, wobei das Amylasesubstrat ein Amylose-Iod-Konjugat umfasst, vorzugsweise ein Stärke-Iod-Konjugat umfasst.

11. Die Vorrichtung nach einem der Ansprüche 1 bis 10, wobei an zumindest einer der Lüftungsöffnungen (16) ein Feuchtigkeitsindikator angebracht ist; vorzugsweise wobei der Feuchtigkeitsindikator an einem luftdurchlässigen Indikatorträger angebracht ist, welcher Indikatorträger an der zumindest einen Lüftungsöffnungen (16) anliegt.

12. Verfahren zur Bestimmung eines Analyten mit einer Vorrichtung nach einem der Ansprüche 1 bis 11, umfassend Aufbringen einer flüssigen Probe in die Vorkammer (4) der Vorrichtung, wobei die flüssige Probe durch das Probekissen (5) aufgesogen wird und durch weitere Sogwirkungen des damit verbundenen Konjugatkissens (8), des damit verbundenen Laufstreifens (11) und des damit verbundenen Saugkissens (14) zumindest teilweise durch das Konjugatkissen (8), durch den Laufstreifen (11), zum Saugkissen (14) befördert wird, wobei in einem Testbereich (12) am Laufstreifen (11) der Analyt gebunden wird.

13. Verfahren nach Anspruch 12, wobei der Analyt mit der flüssigen Probe aufgebracht wird oder wobei der Analyt durch eine Reaktion im Probekissen (5) und/oder im Konjugatkissen (8) oder in einem weiteren Kissen, welches fluidisch dem Laufstreifen vorgereiht ist, aus einer Komponente der flüssigen Probe entsteht.

14. Herstellungsverfahren zur Produktion einer Vorrichtung nach einem der Ansprüche 1 bis 11, umfassend Einbringen des Probekissens (5), des Konjugatkissens (8), des Laufstreifens (11) und des Saugkissens (14) in das Gehäuse (2) mit der Probeneintrittsöffnung (3) und der mindestens einen Lüftungsöffnung (16); wobei das Gehäuse (2) im Bereich der Vorkammer (4) mindestens eine Lüftungsöffnung (16) hat und/oder wobei das Saugkissen ein Amylasesubstrat aufweist.

15. Kit mit einer Vorrichtung nach einem der Ansprüche 1 bis 11 und einem Probenabstrichstab, vorzugsweise mit einem Behälter mit einer Spülflüssigkeit oder Gurgelflüssigkeit.
